# EUROPEAN PATENT APPLICATION

(11) **EP 2 527 423 A1**
(43) Date of publication of application: **28.11.2012**
(21) Application number: 12169177.8
(22) Date of filing: 24.05.2012
(51) Int. Cl.: C12M 3/06

(54) **Dialysis fermenter - biorector with dialysis device**

(30) Priority: 27.05.2011 EP 11167948
(71) Applicant: Roche Diagnostics GmbH, 68305 Mannheim (DE); F. Hoffmann-La Roche AG, 4070 Basel (CH)
(72) Inventor: Hoffmann, Artur, 82515 Wolfratshausen (DE); Lechner, Max, 82377 Penzberg (DE); Reese, Christoph, 81375 Muenchen (DE)
(74) Representative: Burger, Alexander

(57) **Abstract**

The subject matter disclosed herein concerns the use of a semipermeable membrane having a molecular cut-off of 15 kDa to 50 kDa in a dialysis fermenter as a separation layer between (a) the cell-containing liquid culture medium and (b) the non-cell-containing dialysis medium (nutrient solution). Furthermore the disclosed subject matter concerns a dialysis fermenter with compartments for cell-containing culture medium, non-cell-containing nutrient solution as well as an exchange unit of a semipermeable membrane, wherein mass transfer takes place between the culture medium and the dialysis medium by means of diffusion and/or ultrafiltration. The dialysis fermenter includes a functional unit which is suitable for preventing precipitates formed in the dialysis medum from entering the hollow fibers of the exchange unit. Furthermore, a method for culturing cells is disclosed.

## Description

The subject matter disclosed herein concerns the use of a semipermeable membrane having a molecular cut-off of 15 kDa to 50 kDa in a dialysis fermenter as a separation layer between (a) the cell-containing liquid culture medium and (b) the non-cell-containing dialysis medium (nutrient solution). Furthermore the disclosed subject matter concerns a dialysis fermenter with compartments for cell-containing culture medium, non-cell-containing nutrient solution as well as an exchange unit of a semipermeable membrane, wherein mass transfer takes place between the culture medium and the dialysis medium by means of diffusion and/or ultrafiltration. Furthermore, a method for culturing cells is disclosed.

The present invention concerns bioreactors which are understood as devices for culturing biological cells on an industrial scale. The invention provides an improved dialysis fermenter i.e. a bioreactor (fermenter) comprising a first and a second compartment. Culture medium and the biological cells are located in the first compartment and nutrient solution is provided in the second compartment. The operation of the bioreactor (fermentation process) serves on the one hand to propagate and/or culture the cells, on the other hand, the cells produce desired products in this process and secrete these products if necessary into the surrounding culture medium. Mass transfer takes place during the fermentation process between the liquid phases in the first and second compartment by means of diffusion through a semipermeable membrane. This mass transfer is referred to as dialysis and ensures inter alia that undesired metabolic products are transported out of the culture medium. In addition consumed nutrients of the culture medium are replenished from the nutrient solution (dialysis medium) by dialysis. The present invention concerns in particular advantageous properties of certain semipermeable membranes in dialysis.

### Prior Art

The basic design of a dialysis fermenter is described for example in DE 3541738A1 as well as in Comer, M.J. et al., Cytotechnology 3 (1990) 295-299.

DE 3541738A1 discloses a process for culturing cells in which mammalian cells are held in suspension in a culture medium by means of a stirring device in a biorector under controlled conditions and during the fermentation process nutrients are supplied to the cells and waste products of the cells are discharged. The culture medium and cells on the one hand and the nutrient solution on the other hand, are located in two separate compartments. Nutrient solution flows in a flow path along a semipermeable membrane which is permeable to the nutrients and waste products of the cells. The membrane is impermeable to higher molecular components of the culture medium. The culture medium containing the cells is led past one side of the membrane and the nutrient solution is led past the opposite side. Nutrients from the dialysis medium pass through the membrane into the culture medium and in the opposite direction waste products of the cells pass into the nutrient solution. This process is also referred to as dialysis, the nutrient solution is referred to as a dialysis medium and the membrane at the centre of this process is referred to as the dialysis membrane.

Hollow fibres are suitable for dialysis. In US 006/0124531 it is described that hollow fibres are integrated as a bundle in certain embodiments of filter modules.

In dialysis the membranes usually act as a molecular sieve having a certain cut-off which is specified as the largest molecular weight of particles which can still pass the membrane during dialysis. Accordingly molecules having a molecular weight below the cut-off can diffuse through the membrane, whereas larger molecules having a molecular weight above the cut-off are retained by the membrane i.e. they cannot permeate through the membrane. In practical applications the cut-off must be suitably selected so that essential high molecular weight ingredients such as growth factors are not removed from the culture medium into the nutrient solution during dialysis. This would have undesired consequences for cell growth. Since the compositions of cell culture media are often complex especially in the case of mammalian cells, a low cut-off is advantageous.

Hollow fibres which are known under the trade name Cuprophan^{®} (Membrana GmbH, Wuppertal, Germany) are used for current practical applications in dialysis fermentation. Such a hollow fibre (Cuprophan^{®} RC55 8/200 hydrophilic capillary membrane) typically has a wall thickness of 8.4 µm ± 1.1 µm and an inner diameter of 200 µm ± 15 µm and is characterized by a cut-off of about 12 kDa molecular weight. Further properties of Cuprophan^{®} are documented by the manufacturer in the data sheet 456/9095/000 (02/00).

Filter modules consisting of Cuprophan^{®} hollow fibres are in the lower range of the cut-off described in previously published documents. DE 3541738A1 teaches in a general wording that materials such as cellulose acetate, acrylic polymers and polysulfone fibres which have a cut-off (molecular cut-off) at a molecular weight of about 10 kDa to 100 kDa (Da = dalton) are suitable as a dialysis membrane. The same range is disclosed by the publication of Comer M.J. et al. (Cytotechnology 3 (1990) 295-299). It additionally describes a particular ratio of 0.01 m²/1 to 0.3 m²/1 between the membrane surface area and volume of the culture medium. According to DE 3541738A1 a range of 0.03 m²/1 to 0.2 m²/1 is a particular embodiment.

When Cuprophan^{®}-based devices are used for blood dialysis (haemodialyzers) physiological reactions of blood cells with the membrane material may occur (Brandt T. and Wiese F., Contrib. Nephrol. 138 (2003) 1-12; Daveport A., Hemodial. Int. 12 Suppl. 2 (2008) p 29 - p 33). Further information may also be found in the internet (as of 17.03.2011) under the address http://www.dialysemuseum.de/prinzip_membranen.php. For this reason filter modules consisting of Cuprophan^{®} hollow fibres are no longer available on the market even for dialysis fermentation. The problem for a person skilled in the art is now to identify alternative membrane materials for dialysis fermentation. Alternative materials should have improved properties compared to Cuprophan^{®} for use as hollow fibres in filter modules.

It was surprisingly found that membranes with a higher cut-off than that of Cuprophan^{®} have a comparably good or improved effect in dialysis fermentation. Whereas Cuprophan^{®} has a relatively closed surface, it was additionally surprising that materials with larger pores could be advantageously used as a dialysis membrane. Even more surprising was the fact that these advantages were also observed for hollow fibres having a more than 3-fold larger membrane thickness (thickness of the wall formed by the membrane defined as the average shortest path which in the present case separates the compartments of the culture medium and of the dialysis medium) compared to Cuprophan^{®} hollow fibres (8 µm membrane thickness).

### Summary of the Invention

A first subject matter of the invention is therefore the use of a semipermeable membrane having a molecular cut-off of more than 15 kDa to 50 kDA in a dialysis fermenter as a separation layer between (a) the cell-containing liquid culture medium and (b) the non-cell-containing dialysis medium (nutrient solution).

A further subject matter of the invention is a dialysis fermenter comprising a first compartment containing a cell-containing liquid culture medium and a second compartment containing a non-cell-containing dialysis medium (= nutrient solution), additionally comprising an exchange unit (= dialyzer) with a semipermeable membrane which dips into the culture medium, wherein the exchange unit (filter module) is fluidically connected to the second compartment by an inlet as well as an outlet, further comprising a pump which feeds dialysis medium from the second compartment into the exchange unit and from there back again into the second compartment, wherein mass transfer takes place between the culture medium and the dialysis medium along the semipermeable membrane by means of diffusion and/or ultrafiltration, characterized in that the molecular cut-off of the semi-permeable membrane is in a range of 15 kDa to 50 kDa, where 15 kDa and 50 kDa are included in the range.

A further subject matter of the invention is a process for culturing cells in which a cell culture is kept in substantially homogeneous suspension in a culture medium by means of a stirring device under controlled environmental conditions in a biorector (= dialysis fermenter), nutrients for the cells are fed in and waste products of the cells are discharged, wherein a dialysis medium (= nutrient solution) that is separate from the culture medium is used which flows in a flow path which is separated from the culture medium by a semipermeable membrane where the membrane is designed such that it is permeable to the nutrients and the waste products of the cells but is impermeable to higher molecular components of the culture medium and wherein the culture medium containing the cells is led past one side of the membrane and the dialysis medium containing the nutrients is led past the other side of the membrane such that nutrients from the dialysis medium can pass through the membrane into the culture medium and waste products from the culture medium can pass into the dialysis medium, characterized in that the molecular cut-off of the membrane is in the range of 15 kDa to 50 kDa, where 15 kDa and 50 kDa are included in the range.

### Description of the Figures

Each figure contains two diagrams denoted "A" and "B". Each diagram denoted "A" represents data from fermentation 1 using a PES module according to the invention (= exchange unit in the sense of the invention, dialyzer) with a filter located upstream. Each diagram denoted "B" represents data from fermentation 2 using a PES module (= exchange unit) without a corresponding filter. In each diagram the X axis denotes the time in days. The Y axis is divided into relative units in each diagram. In corresponding diagrams "A" and "B" equal relative values also denote equal absolute measured values. If for example a relative value of 0.6 for the parameter live cell density is stated in the respective diagram for fermentation 1 and fermentation 2 this corresponds in both cases to an absolute measured value determined to be of equal magnitude. The same applies to all other parameters listed in the following.
- **Figure 1:**: Live cell density (cells per volume) in the culture medium.
- **Figure 2:**: Product concentration i.e. the concentration in the culture medium of the monoclonal antibody secreted by the hybridoma cells.
- **Figure 3:**: Module input pressure i.e. the pressure in the feed line to the exchange unit. The pressure is generated by the pump which conveys dialysis medium from the second compartment into the exchange unit and from there back again into the second compartment.
- **Figure 4:**: Dialysis flow i.e. the volume of dialysis medium which is pumped per unit of time through the exchange unit.
- **Figure 5:**: Lactate concentration; the solid line denotes the measure values in the culture medium; the line interrupted by white squares denotes the measured values in the dialysis medium.

### Description of the Invention

As used in conjunction with the present invention, semipermeability is the property of a substantial or physical interface. A membrane and in particular a dialysis membrane is a possible embodiment of such an interface. Another embodiment is for example a porous wall. In this connection semipermeability means that only molecules below a certain mole mass or particles below a certain size can migrate through the interface. An interface is permeable for substances which are small enough to migrate through the interface. The permeability of a substance is determined by the cut-off, a property of the interface.

In general it is desirable for the purposes of dialysis fermentation to use semipermeable membranes having cut-offs that are as low as possible. Although 100 kDa is regarded in this connection as a practical upper limit, it can be expected that important substances for the growth of the cell culture such as growth factors with pass into the dialysis medium during the dialysis process and thus leave the culture medium and be no longer available to the cells. Another problem arises in the case of products which are secreted by the cultured cells into the culture medium. During dialysis fermentation it is generally undesired that these products pass into the nutrient solution. At a cut-off of 100 kDa this, however, cannot be excluded in many cases. For this and other reasons, membranes having the lowest possible cut-off have been used up to now.

In the search for substitute materials for Cuprophan^{®} it surprisingly turned out that membranes having a relatively high cut-off (more than 12 kDa) have an equally good or better effect compared to Cuprophan^{®} in dialysis fermentation. A first subject matter of the invention is therefore the use of a semipermeable membrane having a molecular cut-off of more than 15 kDa to 50 kDa (where 15 kDa and 50 kDa are included in the range) in a dialysis fermenter as a separation layer between (a) the cell-containing liquid culture medium and (b) the non-cell-containing dialysis medium.

Suitable dialysis membranes can consist of various materials known to a person skilled in the art. Common membranes consist either of regenerated or modified cellulose or of synthetic materials. The latter include polysulfone (PSU), polyacrylo-nitrile (PAN), polymethylmethacrylate (PMMA), mixtures of polyarylether-sulfones, polyvinylpyrrolidone and polyamide (Polyamix^{®}) and others. The polysulfones include polyethersulfone [poly(oxy-1,4-phenylsulfonyl-1,4-phenyl), abbreviated PES]. Polyethersulfone is advantageous as a semipermeable membrane for the use according to the invention. This may among others be due to the increased hydrophilicity (and/or the improved wettability of the membrane with water) of PES membranes compared to PSU membranes. The wettability of PES membranes can for example be further increased by the inclusion of the water-soluble polymer polyvinylpyrrolidone (Brandt T. and Wiese F. Contrib. Nephrol. 138 (2003) 1-12; DE 3541738; Fujimori, A., Naito, H., Miyazaki, T., Artificial Organs 22 (1998) 1014-1017; Hoenich, N.A., Int. J. Artificial Organs 30 (2007) 964-970).

The dialysis fermenter according to the invention comprises a first compartment containing a cell-containing liquid culture medium and a second compartment containing a non-cell-containing dialysis medium. In addition the dialysis fermenter comprises an exchange unit dipping into the culture medium which is also referred to as a dialyzer. The exchange unit comprises a semipermeable membrane and the exchange unit is fluidically connected to the second compartment by an inlet as well as an outlet. A further component is a pump which conveys dialysis medium from the second compartment into the exchange unit and from there back again into the second compartment. In this process mass transfer between the culture medium and the dialysis medium occurs along the semipermeable membrane in the first compartment by means of diffusion and/or ultrafiltration. The dialysis fermenter according to the invention is in particular characterized in that the molecular cut-off of the aforementioned semipermeable membrane is in the range of 15 kDa to 50 kDa, where 15 kDa and 50 kDa are included in the range.

In an advantageous embodiment of the invention the molecular cut-off of the semipermeable membrane is in the range of 20 kDa to 40 kDa, where 25 kDa and 40 kDa are included in the range. A cut-off of 35 kDa ± 5 % has proven to be particularly practical.

The permeability of a membrane is inversely proportional to the thickness of this membrane. The thickness of the membrane corresponds to the average shortest distance by which the membrane separates the liquid phases of the culture medium and the nutrient solution in the dialyzer. The previously used membranes made of Cuprophan^{®} have a thickness of about 8 µm. Advantageous membranes for the use according to the invention in a dialysis fermenter are characterized by a thickness of about 15 µm to 50 µm; in a particular embodiment the thickness is about 35 µm.

Suitable materials from which the membranes according to the present invention can advantageously be manufactured are polyethersulfone, polysulfone, polyacrylonitrile (PAN), polymethylmethacrylate (PMMA), mixtures of polyarylethersulfone, polyvinylpyrrolidone and polyamide (Polymix^{®}) and other suitable materials known to a person skilled in the art.

The membranes can for example be integrated in the dialyzer as lamellae. However, other forms are also possible. In a particular embodiment the membranes are present as hollow fibres. A hollow fibre is understood as a cylindrical fibre which has one or more continuous hollow spaces in the cross-section. Hollow fibres (hollow fibre synonymous with capillary membrane) are manufactured with semipermeable structures so that the walls of the fibre act as a membrane. The inner diameter of advantageous hollow fibres is 50 µm to 500 µm and their outer diameter is 65 µm to 600 µm. In a particular embodiment, the outer diameter of the hollow fibres is 260 µm ± 10 % and the inner diameter is 190 µm ± 10 %.

In order to construct filter modules, hollow fibres having a typical length between 10 cm and more than 100 cm are combined to form modules having a large filter surface, and are sealed at both ends against hydraulic short circuit. This arrangement is a particular embodiment for the practicing the present teachings. In a particular embodiment, the exchange unit has a cylindrical shape. The degree of filling of the exchange unit is determined by the ratio of the cross-sectional area occupied by hollow fibres to the total cross-sectional area of the cylinder on which the hollow fibres are distributed. In this connection it is advantageous that the degree of filling in the exchange unit is 0.19 to 0.50. It was found that with values within this range a good diffusion of the exchange unit with culture medium is ensured. On the other hand, the exchange area of the hollow fibres is then in an advantageous range. In the case of the degree of filling, a value in the range of about 0.30 to 0.42 has proven to be of particular practical value which is therefore a particular embodiment. A value of about 0.35 can advantageously be chosen for the degree of filling.

Whereas previously an arrangement of hollow fibres in fibre bundles that are separated from one another has been described as a configuration of the exchange unit (US 2006/0124531), a more simple arrangement has proven of value in the concrete case of the dialysis fermenter according to the invention. Correspondingly in a particular embodiment of the exchange unit, the hollow fibres are equally distributed over the cross-sectional area of this exchange unit and are arranged as a bundle that is not further subdivided.

In a specific embodiment the cells are cultured as a suspension in the first compartment of the dialysis fermenter. This means that the cells usually do not adhere to surfaces but are basically uniformly distributed in the culture medium (homogeneous suspension). A stirring unit extending into the first compartment ensures the uniform distribution of the cells during operation.

Theoretically the hollow fibres of the exchange unit can be placed in the culture medium of the first compartment without an additional holder or bundling. However, such an arrangement is impractical because it would make it difficult to remove or replace the hollow fibres in an uncomplicated manner. Furthermore, the individual hollow fibres would be subject to continuous and sometimes strong mechanical loads due to the movement of the stirring unit in the fermenter and the flow of culture medium which this causes. For this reason the exchange unit particularly comprises a cage which surrounds the bundle or bundles of hollow fibres. In this connection it is important that the cage has sufficiently large openings so that there is an adequate flow of culture medium against the hollow fibres in the interior. Conversely the larger the opening of the cage, the higher the packing density of the hollow fibres in the interior of the cage can be.

According to a particular embodiment, the exchange unit is constructed with hollow fibres in a cylindrical shape (cylinder). Shapes other than cylindrical are also possible in the exchange unit. In a particular embodiment in the exchange unit the hollow fibres run basically in a straight line which means that the hollow fibres connect two opposing sides of the cage basically in a straight line. The filling degree of the exchange unit (the packing density of hollow fibres in a ratio to the volume of the exchange unit) can be determined by the ratio of the cross-sectional area occupied by hollow fibres to the total available cross-sectional area over which the hollow fibres can be distributed. In general a degree of filling of 0.19 to 0.50 is advantageous. It is a particular embodiment that the hollow fibres in the exchange unit are basically uniformly distributed. In the concrete case of a cylindrical shape, the ratio of the cross-sectional area occupied by hollow fibres to the total cross-sectional area of the cylinder in which the hollow fibres are distributed is 0.19 to 0.50 in the exchange unit. A degree of filling of approximately 0.35 is in turn a particular embodiment.

In a particular embodiment the exchange unit thus comprises a wall with equally distributed openings or a cage as the outer boundary. The culture medium can flow around the hollow fibres through the openings. In the case of a cylindrical design of the exchange unit, it is a particularly embodiment that the ratio of the free area of the cylinder surface to the total surface is in a range of 0.2 to 0.6. A value of about 0.3 has been shown to be practical.

Mass transfer takes place along the membrane surface in the exchange unit during the fermentation process in which undesired end products of metabolism or metabolic end products that inhibit growth or are toxic in high concentrations pass through the membrane into the dialysis medium. A person skilled in the art selects a suitable ratio of the membrane surface in the exchange unit to the volume of the culture medium which is in contact with the exchange unit in order that mass transfer can take place in a sufficient amount and at a sufficiently high rate. In the case of the use according to the invention of a semipermeable membrane having a molecular cut-off of more than 15 kDa to 50 kDa in a dialysis fermenter, the corresponding ratio [surface (MK)/vol.(K)] of the membrane surface [surface (MK)] facing the cell-containing culture medium to the volume of the culture medium [vol. (K)] is, in a particular embodiment, in the range of 0.1 cm⁻¹ to 1.3 cm⁻¹ and in an other particular embodiment in the range of 0.2 cm⁻¹ to 1.0 cm⁻¹. These values of the ratio are advantageous for volumes of culture medium of the order of magnitude of 101, 100 1, 1000 1 and 2000 1 for dialysis fermenters where each of the said values (x) defines a range with the limits 0.5*x≤ x ≤1.5*x and where the limits are included in the range.

In the dialysis fermenter according to the invention the exchange unit forms the connection between the culture medium in the first compartment and the dialysis medium (nutrient solution) in the second compartment. The nutrient solution is continuously circulated by moving it by means of a pump through a first fluidic connection for example through a rigid pipe or a flexible tube from the second compartment through the hollow fibres of the exchange unit and from there through a further fluidic connection back again into the second compartment. The term "dialysis flow" refers to the volume of dialysis medium which is pumped per unit of time by the exchange unit. In the case of a fermenter containing approximately 100 cell-containing culture medium a particularly advantageous dialysis flow of about 21/min was determined for the previously known material (i.e. Cuprophan^{®} RC55 8/200 hydrophilic capillary membrane having a wall thickness of 8.4 µm± 1.1 µm and an inner diameter of 200 µm ±15 µm as well as a cut-off of about 12 kDa). The pressure-dependent dialysis flow in the case of Cuprophan^{®} is 7 ml/hPa (hPa = hectopascal = mBar). Surprisingly an approximately 140 % - 160 % higher pressure-dependent dialysis flow was found in comparable dialysis fermenters according to the invention (approximately 100 1 cell-containing culture medium). with exchange modules containing hollow fibres made of polyethersulfone. A value measured as an example was 11 ml/hPa. Values for dialysis flow and pressure-dependent dialysis flow of about 9 1/min, 41 ml/hPa (Cuprophan^{®}) or 11.5 1/min, 57.5 ml/hPa (device according to the invention) were found for dialysis fermenters containing approximately 1000 1 cell-containing culture medium.

Depending on various parameters, for example the type of cultured cells, the culture conditions, the age and physiological status of the cell culture, the content of dead cells or cell debris and cell components in the culture medium, or the content of high molecular weight metabolic products in the culture medium, aggregates can sometimes be formed of which some can block the pores of the dialysis membrane. This can take place especially with those aggregates whose total size corresponds approximately to or is smaller than the size of the pores in the membrane. Blocking of membranes during the process of dialysis fermentation is also referred to as "fouling".

Fouling can, on the one hand, occur on the side of the dialysis membrane facing the culture medium. It has proven to be particularly advantageous to specifically reduce the porosity of the dialysis membrane on the side facing the culture medium.

Porosity is a physical quantity and represents the ratio of the volume of void space to the total volume of the membrane material. It can be expressed as the ratio [V_{H}/V] of void space volume [V_{H}] to total volume [V = V_{H} + V_{F}] where [V_{F}] is the pure volume of the solid of the membrane material. Typically and also in the following porosity is stated as a percentage.

In a particular embodiment the cross-section of the semipermeable membrane consists of a first and a second layer where the porosity of the first layer is 30 % to 40 % and the porosity of the second layer is 70 % to 80 %. Both layers advantageously directly adjoin one another and the transition between the two layers can be discrete or continuous i.e. in the latter case without a discrete transition. The width of the first layer is, in a particular embodiment, 1 % to 30 % of the total width of the membrane cross-section. In a special embodiment the first layer faces the culture medium and the second layer faces the dialysis medium. This embodiment can reduce or prevent fouling on the side of the dialysis membrane facing the cell-containing culture medium during the operation of the dialysis fermenter. In another special embodiment the arrangement of the different porous layers is reversed i.e. the first layer in this case faces the dialysis medium and the second layer faces the culture medium.

Fouling of the dialysis membrane on the side facing the dialysis medium can occur when certain substances passing out of the cell culture medium into the nutrient solution exceed threshold concentrations in the nutrient medium. These substances can form aggregates or precipitates when threshold concentrations are exceeded depending on the respective cell culture, its culture medium, the composition of the nutrient medium, the physiological status of the culture, the metabolic products that are formed and the cell components that are released. If these insoluble components enter the hollow fibres their conducting cross-section can be completely blocked.

If small aggregates enter the hollow fibres, they can close the membrane pores and thus impede the mass transfer. An undesired effect of this is that as the fouling increases, the pump has to build up an ever increasing pressure for the inflow into the exchange unit to ensure a constant flow rate. This effect can lead to a cessation of the flow through the exchange unit. In this case metabolic end products are no longer transported out of the culture medium and the cell culture can no longer be supplied and maintained with nutrients by means of dialysis.

In order to avoid such disadvantages, it is very advantageous to connect the inlet lead of the exchange unit to a functional unit which is suitable for preventing precipitates that are formed in the dialysis medium from entering the exchange unit through the inlet. This functional unit can consist of a sieve, a filter, a continuous flow centrifuge or a separation device for sedimenting or floating the precipitate. The said functional unit is placed in front (upstream) of the inlet into the exchange unit on the side of the dialysis medium.

The effects of fouling as described above typically become noticeable after 9 to 10 days at the earliest. This time period may be different and even longer depending on the cultured cell line. For dialysis fermenters according to the invention which are only operated in a time period which is uncritical for fouling, the aforementioned functional unit for separating precipitates is thus not an essential component. The advantageous properties of the membrane used according to the invention are readily realized within the time period and especially the described technical advantages with regard to dialysis flow and pressure.

A filter, in a special embodiment, is provided as a functional unit in the sense that it is suitable for preventing precipitates formed in the nutrient solution from entering the exchange unit through the inlet. A filter is advantageous with a pore size of 2 µm to 0.02 µm, where 2 µm and 0.02 µm are included in the range. A particular pore size is 0.5 µm ± 5 %. A filter, in a particular embodiment, does not limit the dialysis flow and the pressure-dependent dialysis flow through the exchange unit.

The period of use of the exchange unit can be considerably increased by preventing insoluble components in the dialysis medium from coming into contact with the membranes of the hollow fibres in the exchange unit. The duration of the fermentation process itself can also be increased in this manner. Thus, it is possible for example to achieve longer production times of target molecules in cultures in a stationary phase. Examples of target molecules are in particular antibodies and other proteins which are secreted from the interior of cells into the surrounding medium.

A further subject matter of the invention is a process for culturing cells in which a cell culture is kept in substantially homogeneous suspension in a culture medium by means of a stirring device under controlled environmental conditions in a biorector (= dialysis fermenter), nutrients for the cells are fed in and waste products of the cells are discharged, wherein a dialysis medium (= nutrient solution) that is separate from the culture medium is used which flows in a flow path which is separated from the culture medium by a semipermeable membrane where the membrane is designed such that it is permeable to the nutrients and the waste products of the cells but is impermeable to higher molecular components of the culture medium and wherein the culture medium containing the cells is led past one side of the membrane and the dialysis medium containing the nutrients is led past the other side of the membrane such that nutrients from the dialysis medium can pass through the membrane into the culture medium and waste products from the culture medium can pass into the dialysis medium, characterized in that the molecular cut-off of the membrane is in the range of 15 kDa to 50 kDa, where 15 kDa and 50 kDa are included in the range.

The fermentation process according to the invention is in general suitable for culturing all species and types of biological cells which can be kept in suspension culture. Especially suited are insect cells and mammalian cells as well as cell lines derived from mammalian cells. Hybridoma cells are especially suited. Hybridoma cells which secrete monoclonal antibodies are very particularly suited. The process according to the invention particularly takes place in a dialysis fermenter.

The use of a synthetic material as a semipermeable membrane in the exchange unit has a number of advantages compared to cellulose-based membranes (example: Cuprophan^{®}). These advantages are achieved by using polyethersulfone (PES) as an example. Alternative synthetic materials as described here achieve comparable advantageous results.

The following lists individual facts and advantages but not comprehensively:
- Cellulose-based hollow fibres are no longer available in a suitable form on the world market. PES-based hollow fibres are available worldwide.
- The time required to prepare hollow fibre modules containing synthetic hollow fibres before carrying out a fermentation is substantially shorter. Cellulose-based hollow fibres have to be immersed in a preservative solution for storage. This solution contains substances which are not compatible with the culture of eukaryotic cells. Therefore the preservative solution has to be removed by washing. This process is cumbersome and increases time and materials required to prepare fermentation processes. Depending on the hollow fibre module, one has to expect a time requirement of about three hours per module and fermentation. In contrast no pretreatment whatsoever is necessary for polyethersulfone-based modules. The modules can be stored without a preservative and directly incorporated in the fermenter and used as intended.
- Cellulose-based fibres shrink when they are dried. If it is intended to reuse a hollow fibre module manufactured with cellulose-based hollow fibres after fermentation and subsequent cleaning are completed, the hollow fibres have to be immersed in a stabilization solution. Afterwards the hollow fibre module must be heat-sterilized for preservation. These processes are in turn very laborious. Subsequent storage has to take place at 4°C. In contrast PES-based hollow fibre modules can be dried in the air at room temperature after cleaning and can be stored in a dry state at room temperature.
- PES-based hollow fibre modules have a higher pressure-dependent dialysis flow. This allows the pump in the dialysis feed line to have a cheaper design because the pump capacity can be down-scaled.

The following is a list of subject matters which further describe the present invention and represent specific embodiments:
1. Use of a semipermeable membrane having a molecular cut-off of more than 15 kDa to 50 kDa, where 15 kDa and 50 kDa are included in the range, in a dialysis fermenter as a separation layer between (a) the cell-containing liquid culture medium and (b) the non-cell-containing dialysis medium (nutrient solution).
2. Use according to subject matter 1, wherein the molecular cut-off of the semipermeable membrane is in a range of 20 kDa to 40 kDa, where 25 kDa and 40 kDa are included in the range.
3. Use according to subject matters 1 and 2, wherein the molecular cut-off of the semipermeable membrane is 35 kDa ±5 %.
4. Use according to one of the subject matters 1 to 3, wherein the material of the membrane is selected from the group comprising regenerated cellulose, modified cellulose, polysulfone (PSU), polyacrylonitrile (PAN), polymethylmethacrylate (PMMA), polyvinyl alcohol (PVA) and polyarylethersulfone (PAES).
5. Use according to subject matter 4, wherein the material of the membrane is polysulfone and particularly polyethersulfone.
6. Use according to one of the subject matters 1 to 5, wherein the semipermeable membrane consists of a first and a second layer.
7. Use according to subject matter 6, wherein the porosity of the first layer is 30 % to 40 % and that of the second layer is 70 % to 80 %.
8. Use according to subject matter 7, wherein the first and the second layer are directly adjacent and the thickness of the first layer is 1 % to 30 % of the cross-section of the membrane.
9. Use according to subject matter 8, wherein in the exchange unit the first layer of the membrane faces the cell-containing culture medium.
10. Dialysis fermenter comprising a first compartment containing a cell-containing liquid culture medium and a second compartment containing a non-cell-containing dialysis medium (= nutrient solution), further comprising an exchange unit (= dialyzer) with a semipermeable membrane which dips into the culture medium, wherein the exchange unit (filter module) is fluidically connected to the second compartment by an inlet as well as an outlet, further comprising a pump which feeds dialysis medium from the second compartment into the exchange unit and from there back again into the second compartment, wherein mass transfer takes place between the culture medium and the dialysis medium along the semipermeable membrane by means of diffusion and/or ultrafiltration, wherein the molecular cut-off of the semipermeable membrane is in a range of 15 kDa to 50 kDa, where 15 kDa and 50 kDa are included in the range.
11. Dialysis fermenter according to subject matter 10, wherein the first compartment additionally contains a stirring unit, wherein the stirring unit is suitable for keeping the cells in suspension and particularly in an essentially homogeneous suspension.
12. Dialysis fermenter according to one of the subject matters 10 and 11, wherein the membrane in the exchange unit is present as a plurality of hollow fibres arranged in parallel.
13. Dialysis fermenter according to one of the subject matters 10 to 12, wherein the inlet to the exchange unit additionally comprises a functional unit which is suitable for preventing precipitates that are formed in the dialysis medium from entering the hollow fibres of the exchange unit through the inlet.
14. Dialysis fermenter according to subject matter 13, wherein the functional unit is selected from the group comprising a sieve, a filter, a centrifuge and a separation device for sedimenting or floating the precipitate.
15. Dialysis fermenter according to subject matter 14, wherein the functional unit is a filter.
16. Dialysis fermenter according to subject matter 15, wherein the pore size of the filter is 2 µm to 0.02 µm, where 2 µm and 0.02 µm are included in the range.
17. Dialysis fermenter according to subject matter 16, wherein the pore size of the filter is 0.5 µm ± 5 %.
18. Dialysis fermenter according to one of the subject matters 10 to 17, wherein in the exchange unit the ratio [surf. (MK)/vol.(K)] of the membrane surface area [surf. (MK)] facing the cell-containing culture medium to the volume of the culture medium [vol.(K)] is in the range of 0.1 cm⁻¹ to 1.3 cm⁻¹, where 0.1 cm⁻¹ and 1.3 cm⁻¹ are included in the range.
19. Dialysis fermenter according to subject matter 18, wherein in the exchange unit the ratio [surf. (MK)/vol.(K)] of the membrane surface area [surf. (MK)] facing the cell-containing culture medium to the volume of the culture medium [vol.(K)] is in the range of 0.2 cm⁻¹ to 1.0 cm⁻¹, where 0.2 cm⁻¹ and 1.0 cm⁻¹ are included in the range.
20. Dialysis fermenter according to one of the subject matters 12 to 19, wherein the average inner diameter of the hollow fibres in the exchange unit has a value in the range of 50 µm to 500 µm, where 50 µm and 500 µm are included in the range.
21. Dialysis fermenter according to one of the subject matters 12 to 20, wherein the average outer diameter of the hollow fibres in the exchange unit has a value in the range of 65 µm to 600 µm, where 65 µm and 600 µm are included in the range.
22. Dialysis fermenter according to one of the subject matters 20 and 21, wherein in the exchange unit the average outer diameter of the hollow fibres is 260 µm ± 10 % and the average inner diameter of the hollow fibres is 190 µm ± 10 %.
23. Dialysis fermenter according to one of the subject matters 10 to 22, wherein the exchange unit has a cylindrical shape and in the exchange unit the ratio of the cross-sectional area occupied by hollow fibres to the total cross-sectional area of the cylinder over which the hollow fibres are distributed is 0.19 to 0.50, where 0.19 and 0.50 are included in the range.
24. Dialysis fermenter according to subject matter 23, wherein the exchange unit contains openings which are suitable for allowing the culture medium to flow around the hollow fibres and wherein the ratio of the area of the cylinder surface kept open to the total surface of the cylinder is in a range of 0.2 to 0.6, where 0.2 and 0.6 are included in the range.
25. Operation of a dialysis fermenter by employing a use according to one of the subject matters 1 to 9.
26. Carrying out a dialysis fermentation employing a use according to one of the subject matters 1 to 9.
27. Carrying out a dialysis fermentation by means of a dialysis fermenter according to one of the subject matters 10 to 24.
28. Preparing a dialysis fermenter according to one of the claims 10 to 24 employing a use according to one of the subject matters 1 to 9, as well as the result of this preparation.
29. Process for culturing cells using the provided results of subject matter 28 or of a dialysis fermenter according to one of the subj ect matters 10 to 24.
30. Process for culturing cells in which a cell culture is kept in substantially homogeneous suspension in a culture medium by means of a stirring device under controlled environmental conditions in a biorector (= dialysis fermenter), nutrients for the cells are fed in and waste products of the cells are discharged, wherein a dialysis medium (= nutrient solution) that is separate from the culture medium is used which flows in a flow path which is separated from the culture medium by a semipermeable membrane where the membrane is designed such that it is permeable to the nutrients and the waste products of the cells but is impermeable to higher molecular components of the culture medium and wherein the culture medium containing the cells is led past one side of the membrane and the dialysis medium containing the nutrients is led past the other side of the membrane such that nutrients from the dialysis medium can pass through the membrane into the culture medium and waste products from the culture medium can pass into the dialysis medium, characterized in that the molecular cut-off of the membrane is in the range of 15 kDa to 50 kDa, where 15 kDa and 50 kDa are included in the range.
31. Process according to subject matter 30, wherein the biorector is a dialysis fermenter according to one of the subject matters 10 to 24 or a dialysis fermenter as a result of the subject matter 28.
32. Process according to one of the subject matters 30 and 31, wherein the cells are suitable for being kept in a suspension culture.
33. Process according to subject matter 32, wherein the cells are selected from the group comprising insect cells, mammalian cells and cell lines derived from these cells.
34. Process according to subject matter 33, wherein the cells are selected from the group consisting of fusion products, for example hybridoma cells, and transformed cells.
35. Process according to one of the subject matters 30 to 34, wherein the cells produce one or more desired substance(s) and optionally secrete them into the culture medium.
36. Process according to subject matter 35, wherein the molecular weight(s) of the desired substance(s) is/are larger than the molecular cut-off of the membrane (i.e. is larger than a value in the range of 15 kDa to 50 kDa, where 15 kDa and 50 kDa are included in the range).
37. Process according to subject matter 36, wherein the desired substance(s) is/are selected from the group comprising an immunoglobulin, a coagulation factor, a growth factor, a precursor of a signal molecule, for example a peptide hormone, an enzyme, a subunit of a protein complex and fragments or derivatives thereof.
38. Process according to one of of the subject matters 30 to 37, wherein the dialysis medium firstly passes through through a functional unit which is suitable for preventing precipitates, if present in the dialysis medium, before it passes through the membrane into the culture medium.

The following example, the publications mentioned here and the figures elucidate the invention further the scope of protection of which is derived from the patent claims. The described methods are to be understood as examples which still describe the subject matter of the invention even after modifications.

### Example 1

### Comparative dialysis fermentation of a mouse hybridoma cell line

The hybridoma cell line A produces a monoclonal antibody and secretes it into the surrounding liquid culture medium. The antibody as the product can be qualitatively and quantitatively detected in the culture medium by using standard methods. The cell line was cultured in the inventive manner and in particular using according to the invention a semipermeable membrane having a molecular cut-off of 15 kDa to 50 kDa, where 15 kDa and 50 kDa are included in the range, in a dialysis fermenter as a separation layer between (a) the cell-containing liquid culture medium and (b) the non-cell-containing dialysis medium (nutrient solution).

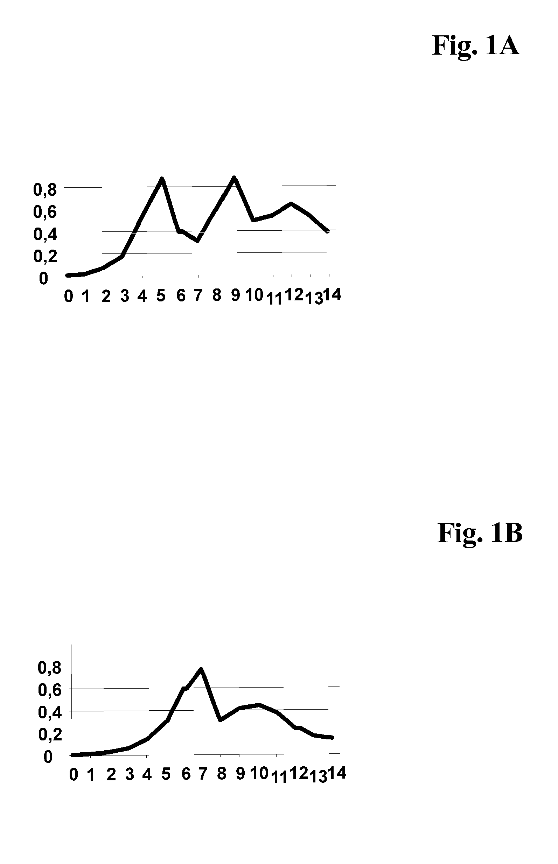

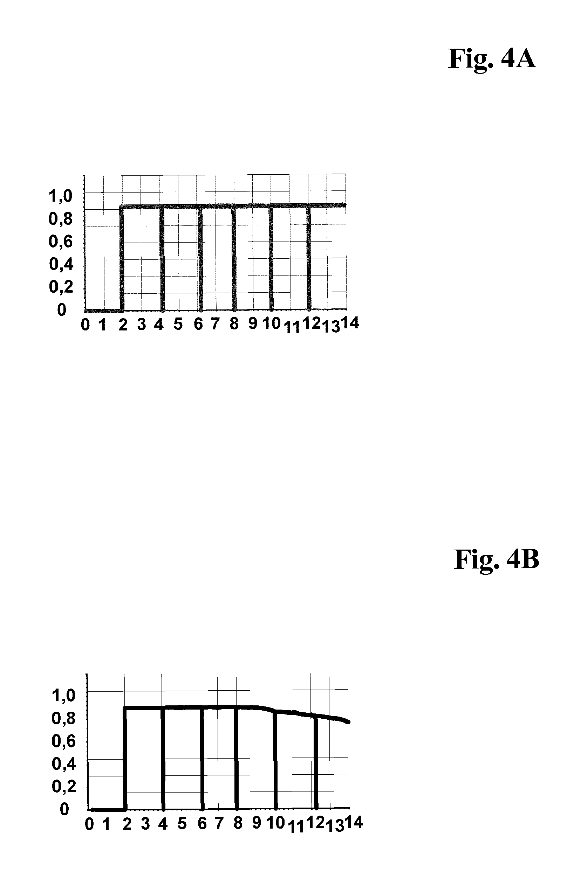

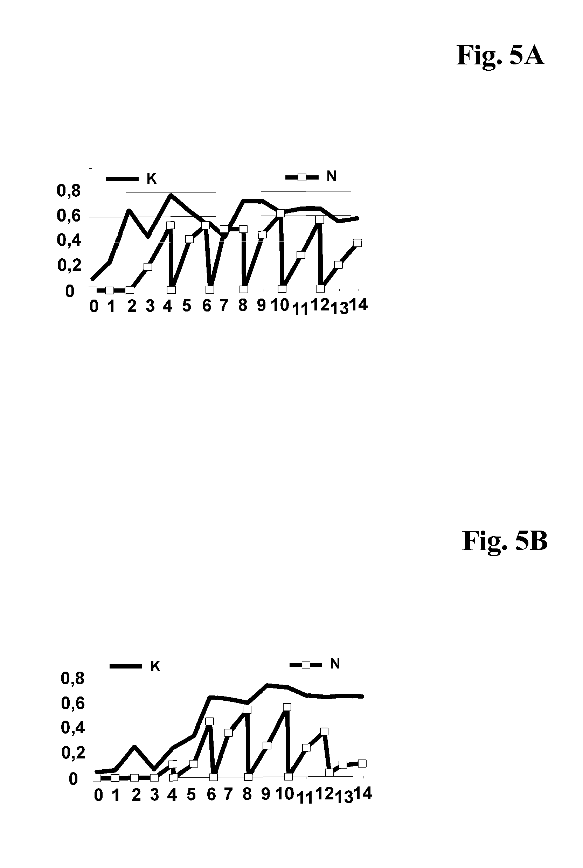

In figures 1 to 5 the course of the fermentation 1 using the mouse hybridoma cell line A is compared with the course of the fermentation 2 using the same hybridoma cell line A in various diagrams. Each figure shows the measured results for a different parameter. Each diagram denoted "A" corresponds to results from fermentation 1; diagrams labelled "B" are those which show the data from fermentation 2.

A principal technical difference between fermentation 1 and fermentation 2 is that in the dialysis fermenter in the case of fermentation 1 the inflow line of the exchange unit is equipped with a functional unit which is suitable for preventing precipitates that are formed in the nutrient solution from entering the exchange unit through the inlet. Otherwise in fermentation 1 and 2 the same exchange units according to the invention were used. These contained PES-based hollow fibres having a molecular cut-off of the semipermeable membrane in a range of about 35 kDa.

The time courses of the live cell density, of the product concentration in the cell-containing culture medium, of the module input pressure (pressure in the inlet to the exchange unit), of the dialysis flow and of the lactate concentration in the culture medium and in the nutrient solution are shown. The dialysis medium was exchanged i.e. replaced by fresh medium in each case after 2, 4, 6, 8, 10 and 12 days. The composition of the dialysis medium was not changed during the processes shown. Both fermentation processes were each terminated after 14 days.

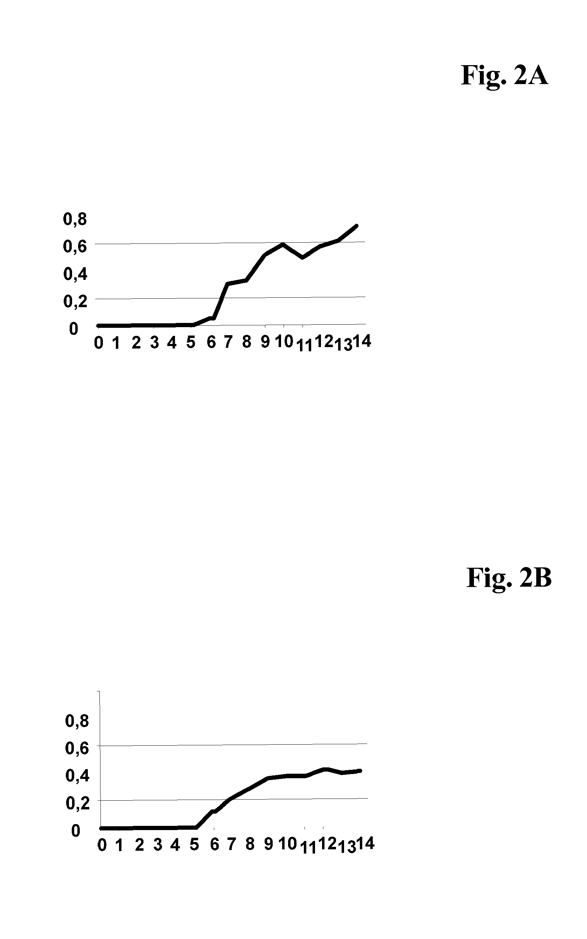

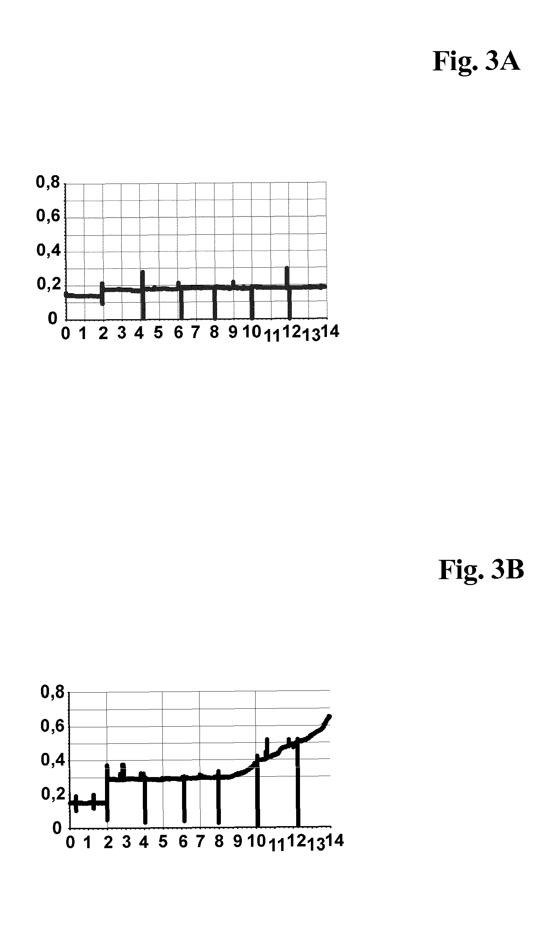

The live cell density decreased in fermentation 2 towards the end of the process compared to fermentation 1 (figure 1). In correlation the product concentration in the dialysis fermenter no longer significantly increased after fermentation day 9 in fermentation 2. In contrast in fermentation 1 the product concentration increased further up to day 14 (figure 2). After about day 9 the inlet pressure on the exchange unit increased continuously in fermentation 2 (figure 3). At the same time the dialysis flow decreased in fermentation 2.

The time course of the lactate concentration in the culture medium and in the nutrient solution was determined as an example as a measure for the mass transfer across the exchange unit. The nutrient solution was replaced in each case after 2 days in the fermentation preparations 1 and 2. This resulted in a continuous increase in the lactate concentration in the nutrient solution until after 2 days the lactate concentration was almost equal in the culture medium and in the nutrient solution. Since mass transfer through the exchange unit was driven especially by the concentration difference of a given substance through the semipermeable membrane, the nutrient solution was exchanged at this time. In the case of fermentation 2 the concentration time courses of lactate in the culture medium and in the nutrient solution showed that the lactate concentration in the nutrient solution remained in each case below the lactate concentration in the culture medium towards the end of the fermentation, after about day 11. In the case of fermentation 1 the lactate concentration in the nutrient solution reached approximately the lactate concentration in the culture medium even towards the end of the fermentation. This effect was particularly surprising and was interpreted to mean that the exchange of lactate by the exchange unit is much better in the case of fermentation 1 than with fermentation 2. An examination showed that in the case of fermentation 2 the exchange unit was blocked by a precipitate. This precipitate had formed in the nutrient solution. The precipitate could also be observed macroscopically in the supply container containing the nutrient solution.

## Claims

1. Dialysis fermenter comprising a first compartment containing a cell-containing liquid culture medium and a second compartment containing a non-cell-containing dialysis medium (= nutrient solution), further comprising an exchange unit (= dialyzer) with a semipermeable membrane, which dips into the culture medium, wherein the exchange unit (filter module) is fluidically connected to the second compartment by an inlet as well as an outlet, and the inlet to the exchange unit additionally comprises a functional unit which is suitable for preventing precipitates that are formed in the dialysis medium from entering the hollow fibres of the exchange unit through the inlet, the dialysis fermenter further comprising a pump which feeds dialysis medium from the second compartment into the exchange unit and from there back again into the second compartment, wherein mass transfer takes place between the culture medium and the dialysis medium along the semipermeable membrane by means of diffusion and/or ultrafiltration, wherein the molecular cut-off of the semipermeable membrane is in a range of 15 kDa to 50 kDa, where 15 kDa and 50 kDa are included in the range.

2. Dialysis fermenter according to claim 1, wherein the first compartment additionally contains a stirring unit and the stirring unit is suitable for keeping the cells in suspension.

3. Dialysis fermenter according to one of the claims 1 and 2, wherein the membrane in the exchange unit is present as a plurality of hollow fibres arranged in parallel.

4. Dialysis fermenter according to one of the claims 1 to 3, wherein in the exchange unit the ratio [surf. (MK)/vol.(K)] of the membrane surface area [surf. (MK)] facing the cell-containing culture medium to the volume of the culture medium [vol.(K)] is in the range of 0.1 cm⁻¹ to 1.3 cm⁻¹, where 0.1 cm⁻¹ and 1.3 cm⁻¹ are included in the range.

5. Dialysis fermenter according to one of the claims 1 to 4, wherein the exchange unit has a cylindrical shape.

6. Dialysis fermenter according to claim 5, wherein the exchange unit contains openings which are suitable for allowing the culture medium to flow around the hollow fibres.

7. Use of a semipermeable membrane having a molecular cut-off of 15 kDa to 50 kDA, where 15 kDa and 50 kDa are included in the range, in a dialysis fermenter according to claim 1 as a separation layer between (a) the cell-containing liquid culture medium and (b) the non-cell-containing dialysis medium (nutrient solution).

8. Use according to claim 7, wherein the material of the membrane is selected from the group comprising regenerated cellulose, modified cellulose, polysulfone (PSU), polyacrylonitrile (PAN), polymethylmethacrylate (PMMA), polyvinyl alcohol (PVA) and polyarylethersulfone (PAES).

9. Process for culturing cells in which a cell culture is kept in substantially homogeneous suspension in a culture medium by means of a stirring device under controlled environmental conditions in a biorector, nutrients for the cells are fed in and waste products of the cells are discharged, wherein a dialysis mediumthat is separate from the culture medium is used which flows in a flow path which is separated from the culture medium by a semipermeable membrane where the membrane is designed such that it is permeable to the nutrients and the waste products of the cells but is impermeable to higher molecular components of the culture medium and wherein the culture medium containing the cells is led past one side of the membrane and the dialysis medium containing the nutrients is led past the other side of the membrane such that nutrients from the dialysis medium can firstly pass through a functional unit which is suitable for preventing precipitates, if present in the dialysis medium, and secondly pass through the membrane into the culture medium and waste products from the culture medium can pass into the dialysis medium, wherein the molecular cut-off of the membrane is in the range of 15 kDa to 50 kDa, where 15 kDa and 50 kDa are included in the range.

10. Process according to claim 9, wherein the cells are suitable for being kept in a suspension culture.

11. Process according to one of the claims 9 and 10, wherein the cells produce a desired substance and optionally secrete said substance into the culture medium.

12. Process according to claim 11, wherein the molecular weight of the desired substance(s) is larger than the molecular cut-off of the membrane.

13. Process according to claim 12, wherein the desired substance is selected from the group comprising an immunoglobulin, a coagulation factor, a growth factor, a precursor of a signal molecule, an enzyme, a subunit of a protein complex and fragments or derivatives thereof.

14. Process according to one of the claims 9 to 13, wherein the cells are selected from the group comprising insect cells, mammalian cells and cell lines derived from these cells.

15. Process according to one of the claims 9 to 14, wherein the cells are selected from the group comprising hybridoma cells and transformed cells.
